# EUROPEAN PATENT SPECIFICATION

(11) **EP 3 217 958 B1**
(45) Date of publication and mention of the grant of the patent: **26.02.2020**
(21) Application number: 15794158.4
(22) Date of filing: 11.11.2015
(51) Int. Cl.: A61K 31/495, A61K 31/4745, A61K 31/205, A61K 31/282, A61K 31/704, A61K 31/337, A61K 38/17, A61K 9/00, A61K 9/16, A61K 9/51, A61K 47/34, A61K 47/50, A61P 5/00, A61P 25/00, A61P 27/02

(54) **SUSTAINED RELEASE ENCAPSULATED NANOPARTICLES**
VERKAPSELTE NANOPARTIKEL MIT VERZÖGERTER FREISETZUNG
NANOPARTICULES ENCAPSULÉES À LIBÉRATION PROLONGÉE

(30) Priority: 11.11.2014 GB 201420080
(43) Date of publication of application: 20.09.2017
(73) Proprietor: Midatech Ltd, Milton Park, Milton Abingdon Oxfordshire OX14 4RD (GB); Midatech Pharma (Wales) Limited, Cardiff, South Glamorgan CF24 0AA (GB)
(72) Inventor: WILLIAMS, Phillip, Abingdon Oxfordshire OX14 4RQ (GB); GROVES, Rhian, Cardiff South Glamorgan CF24 0AA (GB); PALMER, Daniel, Cardiff CF24 4JH (GB)
(74) Representative: Mewburn Ellis LLP
(86) International application number: PCT/EP2015/076364
(87) International publication number: WO 2016/075211

(56) References cited:
- WO-A1-2010/052503
- WO-A1-2010/059135
- WO-A1-2011/119881
- WO-A1-2011/156711
- WO-A1-2012/042273
- US-A1- 2014 220 135
- REN TIANTIAN ET AL: "Sustained-release polylactide-co-glycolide microspheres loaded with pre-formulated protein polysaccharide nanoparticles", MICRO AND NANO LETTERS, THE INSTITUTION OF ENGINEERING AND TECHNOLOGY, GB, vol. 6, no. 2, 28 February 2011 (2011-02-28), pages 70-74, XP006037770, ISSN: 1750-0443, DOI: 10.1049/MNL:20100107

## Description

### Field of the invention

The present invention relates to nanoparticles encapsulated within polymeric microparticles, particularly for use in medicine, and includes methods for treatment of disorders. Pharmaceutical compositions, processes for production of the microparticles and methods for their use are also disclosed.

### Background to the invention

The present invention is directed at compositions and products, and methods of making and administering such compositions and products, including for the treatment of mammals and particularly humans.

Drug delivery poses several significant challenges, particularly with regard to the site and duration of action. In the case of treatment of certain tumours, for example, there remains a need for delivery systems that are able to target anti-cancer drugs to the tumour site and which are able to sustain an efficacious drug concentration over an extended period ideally while minimising the need for frequent repeat drug administration.

Ren et al., Micro & Nano Letters, 2011, Vol. 6, No. 2, pp. 70-74, describes sustained release polylactide-co-glycolide microspheres loaded with pre-formulated protein polysaccharide nanoparticles.

WO2011/119881 describes multi-compartmental nanoparticulate systems for imaging as well as the diagnosis, monitoring and treatment of inflammation and/or disease.

WO2010/059135 describes microparticles have a width of at least about 1 micron and which include a biocompatible polymer, a nanoparticle and an anti-cancer agent.

US2014/0220135 describes permeation enhanced active-carrying nanoparticles, particularly for use in medicine.

WO2010/052503 describes the use of nanoparticles for imaging a tumour in a mammal using electrical impedance tomography.

WO2012/042273 and WO2012/042274 describe apparatus and process for the preparation of solid beads that encapsulate a bioactive agents and which are suitable for use in sustained release, e.g., via depot injection.

WO2013/042125 describes double nano-encapsulation for protecting and controlling the release of active agents, either hydrophobic or hydrophilic, from stable nanoparticles of opposite characteristics.

WO2011/154711 describes glycated gold nanoparticles that act as carriers for delivery of peptides such as insulin.

WO 2011/156711 and WO2012/170828 describe therapeutic or bioaffecting film delivery systems in which nanoparticles having actives bound to or associated therewith are incorporated within a polymeric film matrix.

WO2014/125256 describes nanoparticle delivery systems for use in targeting biologically active agents to the central nervous system (CNS), e.g., for treatment of CNS disorders.

There remains an unmet need for further nanoparticle delivery systems and for methods of delivering such bioactive agents to a subject at the appropriate location and/or for the appropriate duration. The present invention addresses these and other needs.

### Brief Description of the Invention

Broadly, the present invention relates to nanoparticle-drug conjugates encapsulated in micron-scale beads as defined in the claims. The present inventors have surprisingly found that it is possible to encapsulate metal- (e.g. gold-) core nanoparticles within biocompatible polymeric microparticles with high entrapment efficiency.

Accordingly, in a first aspect the present invention provides a microparticle comprising at least one biocompatible polymer, the microparticle encapsulating at least one nanoparticle, the nanoparticle comprising:
(i) a core comprising a metal and/or a semiconductor;
(ii) a corona comprising a plurality of ligands covalently linked to the core, wherein said ligands comprise at least one carbohydrate and/or glutathione,
wherein the microparticle has a diameter along its longest dimension that is within the range 10 µm to 75 µm.

In accordance with this and other aspects of the present invention, the at least one nanoparticle preferably further comprises at least one biologically active agent (e.g. a pharmaceutical active as a "payload") and/or a detectable label (e.g. a fluorophore). However, it has been found that certain nanoparticles exhibit biological activity even in the absence of an additional active agent. Therefore, it is specifically contemplated herein that the nanoparticle need not necessarily have any added active agent. When the nanoparticle does comprise said biologically active agent the agent is generally associated with the core and/or corona of the nanoparticle. Conveniently this association may be in the form of a covalently link, e.g., via a linker group attached to the nanoparticle core or to one or more of the nanoparticle ligands.
For example, an alkyl and/or glycol linker may connect an agent such as a chemotherapeutic agent to the nanoparticle core. In certain cases the active agent may be non-covalently bound to said corona and/or said core. For example, the active agent may comprise a peptide or polypeptide bound non-covalently (e.g. via electrostatic interaction or Van der Waals forces or otherwise) to the ligands that make up the corona of the nanoparticle.

In certain cases the at least one biologically active agent may comprise a chemotherapeutic agent and/or may be covalently linked to said core via a linker.

In certain cases the at least one biologically active agent may be covalently linked to said core via a linker comprising C2-C15 alkyl and/or C2-C15 glycol.

In certain cases the biologically active agent comprises a chemotherapeutic or "anti-cancer" agent, which may preferably be selected from the group consisting of: temozolomide, irinotecan, chlorotoxin, carmustine, platinum(IV), platinum(II), camptothecin, doxorubicin, docetaxel, Maytansine, Maytansinoids, monomethyl auristatin E (MMAE) and histone deacetylase (HDAC) inhibitors such as Panobinostat, Vorinostat, Romidepsin and Chidamide.

In certain cases the nanoparticle further comprises a cell, tissue or tumour targeting moiety. Owing to the advantageous avidity effects of the multiple ligands presented by the nanoparticle, it has been found that a number of targeting moieties are capable of targeting the nanoparticles described herein to certain sites of interest, e.g., tumour or diseased tissue sites, organ types or particular cell types. In certain cases, the targeting moiety may be selected from the group consisting of: folic acid, lactose, albumin, glutamine, a cell surface receptor binding ligand (e.g. an EGFR ligand such as Gefitinib) and an antibody, particularly an antibody that binds selectively to a tumour associated antigen.

In some cases the at least one biologically active agent comprises at least one peptide or polypeptide that is non-covalently bound to the corona. For example, the peptide or polypeptide may be selected from the group consisting of: insulin, GLP-1, amylin, exenatide, octreotide, teriparatide, glucagon, a cytokine, and an antibody.

In some cases the corona of the nanoparticle comprises at least 5, 10, 20 or at least 50 ligands per nanoparticle core. The corona may, in certain cases, be a mixed corona. For example, the corona may comprise two or more different species of ligand (e.g., carbohydrate and non-carbohydrate, such as alpha-galactose and PEGamine; glutathione and carbohydrate; two different saccharides such as glucose and galactose or N-acetyl glucosamine and lactose). The ratio of the first species of ligand to the second species of ligand may be in the range 1:100 to 100:1, preferably 80:20 to 20:80, yet more preferably 60:40 to 40:60).

In some cases the number of molecules of said biologically active agent per nanoparticle core is selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30.

In some cases the diameter of the core of the nanoparticle is in the range 1 nm to 5 nm. The diameter of the nanoparticle including its ligands may in some cases be in the range 2 nm to 50 nm, or 3 nm to 30 nm, or 4 nm to 20 nm, or 5 nm to 15 nm.

In some cases the core of the nanoparticle comprises a metal selected from the group consisting of: Au, Ag, Cu, Pt, Pd, Fe, Co, Gd, Eu and Zn, or any combination thereof. In certain cases the nanoparticle has a core comprising or consisting of gold atoms.

The microparticle of this and other aspects of the present invention typically has more than one, e.g., more than 10, 100, 1000, 10000, 100000, 10⁶, 10⁷ or more than 10⁸ individual nanoparticles encapsulated therein.

In certain cases the microparticle comprises a plurality of said nanoparticles, wherein at least 10%, 20%, 30%, 40%, 50%, 60%, 70%, 80%, 90% or 95% of the nanoparticles in said microparticle have at least one biologically active agent conjugated or bound thereto.

In certain cases the microparticle comprises at least two different species of biologically active agent. Preferably, the at least two different species of biologically active agent work in concert to potentiate or otherwise enhance the biological or therapeutic activity in comparison to either one species alone. Examples specifically contemplated herein are insulin and GLP-1, two or more chemotherapeutic agents, a chemotherapeutic agent in combination with an inhibitor of anti-cancer drug resistance.

In certain cases the at least two difference species of biologically active agent comprise a first and a second agent. In some cases the first biologically active agent is conjugated or bound to a first nanoparticle and a second of said at least two different species of biologically active agent is conjugated or bound to a second nanoparticle. The two or more nanoparticles may then advantageously be present within a single microparticle (i.e. two different nanoparticles). Alternatively or additionally, the at least one nanoparticle may have at least two different species of biologically active agent conjugated or bound thereto (i.e. a combination payload nanoparticle).

The microparticle has a diameter along its longest dimension that is within the range 10 µm to 75 µm. In some cases the morphology of the microparticle is substantially spherical. Without wishing to be held to any particular theory, the present inventors believe that the spherical shape affords greater structural resistance, e.g., resistance to pressure differentials. In one particularly contemplated form the microparticle is spherical and comprises nanoparticles conjugated to a chemotherapeutic such as temozolomide or carmustine. Such microparticles may find use in the treatment of brain tumours, including secondary tumours, in which the microparticles are implanted following or at the time of surgical resection, e.g. to prevent or inhibit cancer recurrence. Owing to their structurally more robust shape, the spherical microparticles are expected to overcome shortcomings that have been seen with *GLIADEL Wafer* in this therapeutic context. However, other non-spherical shapes are specifically contemplated herein. In certain cases the microparticle of the present invention may be relatively flattened or disc-shaped, e.g., the microparticle morphology may be lenticular.

In certain cases the surface porosity of the microparticle of the present invention may be characterised by an average surface pore size of <20 nm, <100 nm, < 1 µm or < 10 µm. As described in WO2012/042274, certain process conditions such as the temperature of the second liquid and/or the pH of the second liquid (e.g. pH 3 to 9) .

In accordance with this and other aspects of the present invention the at least one biocompatible polymer may be any polymer disclosed in WO2012/042274. In certain cases the polymer may be selected from the group consisting of: a polylactide, a polycaprolactone, a polyanhydride and a co-polymer of lactic acid and glycolic acid ("PLGA"). The weight average molecular weight (MW) of the polymer may be from 4 to 700 kDaltons, preferably 4 to 120 kDaltons, more preferably from 4 to 15 kDaltons. Polymer end groups and inherent viscosities may, in certain cases, be as set forth in WO2012/042274. In certain cases the polymer comprises at least one end group or terminal functional group selected from the group consisting of: carboxyl, hydroxyl and ester.

Without wishing to be bound by any particular theory, the present inventors believe that the encapsulation efficiency of nanoparticles within the microparticle and/or the rate or extent of nanoparticle release from the microparticle, e.g. in use, may be influenced (increased or decreased) by virtue of interactions between the nanoparticle corona and the polymeric matrix, e.g., through the selection of a polymer end-group that binds to a functional group present on one or more of the ligands making up the nanoparticle corona. One example of this contemplated herein is a microparticle wherein the at least one biocompatible polymer comprises a co-polymer of lactic acid and glycolic acid ("PLGA") having a carboxylate end group and said nanoparticle corona comprises at least one amine group. In particular, the nanoparticle may comprise a corona of PEGamine ligands, such as 1-amino-17-mercapto-3,6,9,12,15,-pentaoxa-heptadecanol, and alpha-galactose ligands, such as 2-thio-ethyl-α-D-galactoside. Accordingly, in certain cases of the present invention the polymer or co-polymer comprises at least one end group or terminal functional group that binds to a functional group present on the corona of said nanoparticle.

In certain cases in accordance with the present invention the at least one biocompatible polymer may have a ratio of lactide to glycolide ("L/G ratio") in the range 1:1 to 1:0, optionally having a ratio of lactide to glycolide in the range 70:30 to 90:10. The L/G ratio is thought to influence the rate of microparticle breakdown *in vivo* and therefore to influence the duration of release of nanoparticles and/or actives from the microparticle

In certain cases the nanoparticles are distributed substantially homogeneously throughout the microparticle. For example, the density of nanoparticles measured in terms of number of nanoparticles per unit volume of the microparticle may, in certain cases, not vary by more than 50%, more than 20% or more than 10% about the mean. Distribution of nanoparticles within the microparticle may, for example, be determined by electron microscopy. Nanoparticles may be visualised and counted and, if necessary, statistical analyses applied to compute a measure of nanoparticle distribution homogeneity.

In certain cases the nanoparticles have a distribution within the microparticle that is biased towards the surface of the microparticle. For example, more than 50%, more than 80% or more than 90% of the nanoparticles may be located closer to the surface of the microparticle than they are to the centre of the microparticle. As above, the distribution of nanoparticles within the microparticle may, for example, be determined by electron microscopy. Nanoparticles may be visualised and counted and, if necessary, statistical analyses applied to compute a measure of nanoparticle distribution heterogeneity.

In certain cases, the average concentration of nanoparticles expressed in terms of % weight of nanoparticle per weight of microparticle polymer (w/w) is within the range 0.01% to 25% w/w, optionally in the range 0.05% to 10% w/w.

In certain cases, the average concentration of biologically active agent, where present, expressed in terms of % weight of agent per weight of microparticle polymer is within the range 0.005% to 20% w/w, e.g., 0.01% to 10% w/w.

As described herein, microparticles of the present invention advantageously provide sustained release in clinical use. In certain cases the *in vivo* release profile (i.e. release of nanoparticles, particularly nanoparticles having a pharmaceutical payload, from the microparticle) of the microparticle following depot injection of the microparticle in a mammalian subject is in the range 1 week to 6 months, optionally in the range 3 weeks to 3 months. Release profile can be determined according to *in vivo* experimental techniques (e.g. animal models) known to those skilled in the art and/or can be modelled using one or more *in vitro* assays of drug release. Preferably the release profile is such that the rate of release of the nanoparticles into the body (e.g. cellular or extracellular space) is substantially slower than the rate of release of nanoparticles in the absence of encapsulation (e.g. a suspension of nanoparticles injected directly). In preferred cases, substantially slower in this context means at least 10 times, 20 times, 50 times or 100 times slower.

In a second aspect the present invention provides a pharmaceutical composition comprising a plurality of microparticles of the first aspect of the invention and a pharmaceutically acceptable carrier or excipient.

In some cases the pharmaceutical composition comprises an anti-agglomerating agent. The anti-agglomerating agent may take the form of a coating applied to the microparticles, e.g., mannitol.

In some cases the carrier is an aqueous or organic liquid, such as a biocompatible oil. In some cases the pharmaceutical composition takes the form of a lyophilised powder of microparticles suitable for reconstitution, e.g., prior to use.

In certain cases the pharmaceutical composition may be formulated for delivery via an injectable route, such as subcutaneous or intramuscular depot injection, or formulated for implantation during surgery.

In a third aspect the present invention provides a microparticle of the first aspect of the invention or a pharmaceutical composition of the second aspect of the invention for use in medicine wherein the at least one nanoparticle encapsulated by the microparticle further comprises at least one biologically active agent comprising a drug or pro-drug that exerts a therapeutic effect.

In a fourth aspect the present invention provides a microparticle of the first aspect or a pharmaceutical composition of the second aspect for use in a method of treatment of a cancer in a mammalian subject, wherein said microparticle comprises a chemotherapeutic agent.

In some cases the microparticle comprises an chemotherapeutic agent and the microparticle or composition is for treatment of a cancer selected from the group consisting of: a brain cancer, such as a glioblastoma, glioma or astrocytoma; an ovarian cancer; a liver cancer; and a skin cancer. In some cases the chemotherapeutic agent is selected from the group consisting of: temozolomide, irinotecan, chlorotoxin, carmustine, platinum(IV), platinum(II), camptothecin, doxorubicin, docetaxel, Maytansine, Maytansinoids, monomethyl auristatin E (MMAE) and histone deacetylase (HDAC) inhibitors such as Panobinostat, Vorinostat, Romidepsin and Chidamide.

In accordance with the microparticle or pharmaceutical composition for use of the fourth aspect of the invention, the time interval between administration of doses may be at least 1 week, such as at least 2, 3, or 4 weeks, and preferably at least 1, 2, 3, 4, 5 or 6 months.

In a fifth aspect the present invention provides an article of manufacture comprising:
a microparticle of the first aspect of the invention or a pharmaceutical composition of the second aspect of the invention;
a container for housing the microparticle or pharmaceutical composition; and
an insert and/or label.

In certain cases the insert and/or label provides instructions, dosage and/or administration information relating to the use of the microparticle or pharmaceutical composition in a cancer in a mammalian subject.

In a sixth aspect the present invention provides a process for producing a microparticle of the first aspect of the invention, the process comprising:
providing a first liquid comprising a solute, a solvent and a plurality of nanoparticles intended to be encapsulated within the microparticles, the solute comprising a polymer, the concentration of polymer in the first liquid being at least 10% w/v, 'w' being the weight of the polymer and 'v' being the volume of the solvent, the nanoparticles comprising:
(i) a core comprising a metal and/or a semiconductor; and (ii) a corona comprising a plurality of ligands covalently linked to the core, wherein said ligands comprise at least one carbohydrate and/or glutathione;
   providing a liquid droplet generator comprising a piezoelectric component operable to generate liquid droplets,
   causing the liquid droplet generator to form liquid droplets of the first liquid;
   passing the liquid droplets through a gas,
   contacting the liquid droplets with a second liquid so as to cause the solvent to exit the droplets, thus forming solid microparticles;
   the solubility of the solvent in the second liquid being at least 5g of solvent per 100ml of second liquid, the solvent being substantially miscible with the second liquid,
   wherein the second liquid is provided as a flow and the method comprises contacting the liquid droplets with the flow of second liquid.

In certain cases the nanoparticles are provided in an organic solvent:water mixed phase, optionally a DMSO:water mixed phase, and further optionally a DMSO:water mixed phase having a DMSO:water ratio within the range 80:20 to 95:5. Biocompatible nanoparticles as described herein are typically provided in the form of an aqueous suspension. The present inventors have surprisingly found that it is possible to prepare nanoparticles, such as gold core glutathione or glycated nanoparticles, in a DMSO-mixed water phase, e.g., 90:10 DMSO:water. The DMSO:water mixed phase provides certain advantageous compatibility with the microparticle production process, e.g., as described in WO2012/042274. In particular, this addresses the problem that polymer such as PLGA is insoluble in water. Therefore, use of high concentrations of nanoparticles provided in a mixed phase has particular suitability for use in the process of this aspect of the present invention.

In certain cases the first liquid comprises DMSO, poly-lactide-co-glycolide co-polymer ("PLGA") and nanoparticles, the nanoparticles being as defined in connection with the first aspect of the present invention.

In certain cases the second liquid may be as defined in WO2012/042274. In certain cases the second liquid comprises water. The second liquid may comprise a water-alcohol mix (e.g. having a water:alcohol ration in the range 95:5 to 75:25). In particular cases the second liquid may comprise a mixture of water and tert-butanol (e.g. 85:15 water:tert-butanol).

In accordance with this aspect of the present invention the second liquid may have a temperature as described in WO2012/042274. In certain cases, the temperature of the second liquid may be within the range 1°C to 10°C, such as 4°C to 8°C.

In some cases the concentration of polymer in the first liquid may be at least 20%, at least 30% or at least 40% w/v.

In some cases the process of this aspect of the present invention employs an apparatus as described in WO2012/042274. In some cases the process of this aspect of the present invention employs an apparatus as described in WO2012/042273.

In some cases the process further comprises collecting the solid microparticles by separating the solid microparticles from the second liquid, optionally under vacuum. In certain cases, the step of collecting the microparticles employs a bead collection device as described in WO2013/014466. The method of separating the solid microparticles may be as described in WO2013/014466.

In some cases the process further comprises collecting the solid microparticles and formulating or packaging the microparticles into a pharmaceutical composition or delivery form. This may, in certain cases, comprise freeze-drying the microparticles and/or subjecting the microparticles to a sterilization step. In some cases the microparticles may be formulated into a liquid for delivery by depot injection.

In some cases the process of this aspect of the invention further comprises a preceding stage in which the nanoparticles are produced, the preceding stage comprising:
combining a solution comprising glutathione and/or a sulphur derivatised carbohydrate (e.g. a thioalkyl or thioglycol linker attached to a sugar group such as a monosaccharide, disaccharide or polysaccharide) with a solution comprising a core-forming material (e.g. a gold salt) and with a reducing agent (e.g. sodium borohydride), thereby causing the nanoparticle to self-assemble.

In certain cases the preceding stage of producing the nanoparticles further comprises conjugating or binding said biologically active agent to the nanoparticle. Where conjugation involves a covalently attached linker that conjugates the active to the nanoparticle this is advantageously achieve during the self-assembly process. In particular, the preceding stage of producing the nanoparticles may comprise providing a sulphur derivatised linker (e.g. thioalkyl, thioglycol or thioalkylpolyglycol) covalently attached to the agent and combining:
the solution comprising the sulphur derivatised linker covalently attached to the agent;
the solution comprising glutathione and/or a sulphur derivatised carbohydrate;
the solution comprising a core-forming material; and
the reducing agent. The sulphur derivatised compounds are typically provided in oxidised form as disulphides which allows the self-assembly process to proceed upon addition of the reducing agent.

In some cases the sulphur derivatised linker covalently attached to the agent comprises thio alkyl and/or thio glycol linker covalently linked to the agent.

In some cases, particularly where the biologically active agent comprises a peptide or polypeptide, the nanoparticle may be produced first and brought into contact with a solution comprising the peptide or polypeptide and the peptide or polypeptide allowed to bind non-covalently to the nanoparticle corona.

In accordance with all aspects of the present invention the microparticles and methods for their production may be sterile. Additionally or alternatively, the nanoparticles and methods for their production may be sterile. However, it is specifically contemplated herein that the production of the nanoparticles and/or the production of the microparticles includes one or more sterilization steps.

In accordance with the present invention, particularly the fourth aspect thereof, the subject may be a human, a companion animal (e.g. a dog or cat), a laboratory animal (e.g. a mouse, rat, rabbit, pig or non-human primate), a domestic or farm animal (e.g. a pig, cow, horse or sheep). Preferably, the subject is a human.

The present invention includes the combination of the aspects and preferred features described except where such a combination is clearly impermissible or is stated to be expressly avoided. These and further aspects and embodiments of the invention are described in further detail below and with reference to the accompanying examples and figures.

### Brief Description of the figures

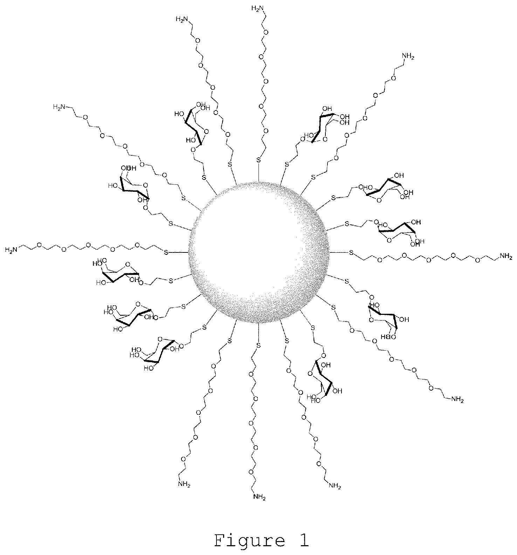

**Figure 1** shows a schematic representation of a nanoparticle having a plurality of ligands in the ratio of 1:1 of alpha-galactose:PEGamine and a gold core.

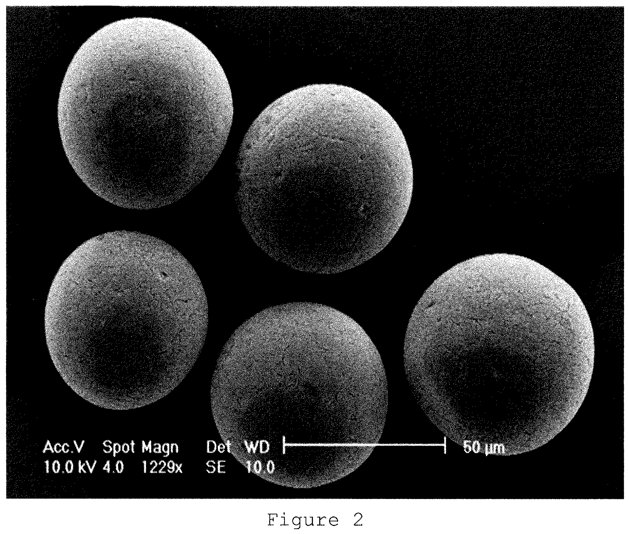

**Figure 2** shows an electron microscopy image of spherical microparticle. A 50 µm scale bar is shown.

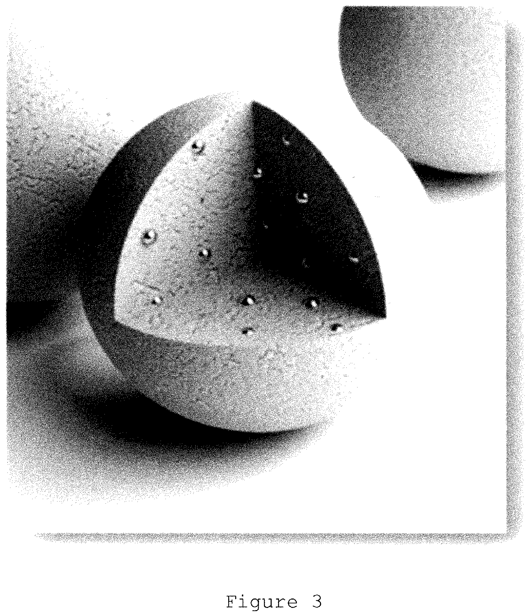

**Figure 3** shows a schematic "cut-away" representation of a microparticle of the present invention. The nanoparticles encapsulated within the microparticle are shown (not to scale).
**Figure 4** shows an electron micrograph of microparticles produced as described in experiment 1 of Example 3. The scale bar shows 50 µm.
**Figure 5** shows a standard curve of absorbance at 382 nm against gold concentration in µg/ml.

### Detailed description of the invention

In describing the present invention, the following terms will be employed, and are intended to be defined as indicated below.

### Nanoparticles

As used herein, "nanoparticle" refers to a particle having a nanomeric scale, and is not intended to convey any specific shape limitation. In particular, "nanoparticle" encompasses nanospheres, nanotubes, nanoboxes, nanoclusters, nanorods and the like. In certain embodiments the nanoparticles and/or nanoparticle cores contemplated herein have a generally polyhedral or spherical geometry.

Nanoparticles comprising a plurality of carbohydrate-containing ligands have been described in, for example, WO 2002/032404, WO 2004/108165, WO 2005/116226, WO 2006/037979, WO 2007/015105, WO 2007/122388, WO 2005/091704 and such nanoparticles may find use in accordance with the present invention.

As used herein, "corona" refers to a layer or coating, which may partially or completely cover the exposed surface of the nanoparticle core. The corona includes a plurality of ligands which generally include at least one carbohydrate moiety, one surfactant moiety and/or one glutathione moiety. Thus, the corona may be considered to be an organic layer that surrounds or partially surrounds the metallic core. In certain embodiments the corona provides and/or participates in passivating the core of the nanoparticle. Thus, in certain cases the corona may include a sufficiently complete coating layer substantially to stabilise the semiconductor or metal-containing core. However, it is specifically contemplated herein that certain nanoparticles having cores, e.g., that include a metal oxide-containing inner core coated with a noble metal may include a corona that only partially coats the core surface. In certain cases the corona facilitates solubility, such as water solubility, of the nanoparticles of the present invention.

Nanoparticles are small particles, e.g. clusters of metal or semiconductor atoms, that can be used as a substrate for immobilising ligands.

Preferably, the nanoparticles have cores having mean diameters between 0.5 and 50nm, more preferably between 0.5 and 10nm, more preferably between 0.5 and 5nm, more preferably between 0.5 and 3nm and still more preferably between 0.5 and 2.5nm. When the ligands are considered in addition to the cores, preferably the overall mean diameter of the particles is between 2.0 and 20 nm, more preferably between 3 and 10 nm and most preferably between 4 and 5 nm. The mean diameter can be measured using techniques well known in the art such as transmission electron microscopy.

The core material can be a metal or semiconductor (said semiconductor optionally comprising metal atoms or being an organic semiconductor) and may be formed of more than one type of atom. Preferably, the core material is a metal selected from Au, Fe or Cu. Nanoparticle cores may also be formed from alloys including Au/Fe, Au/Cu, Au/Gd, Au/Fe/Cu, Au/Fe/Gd and Au/Fe/Cu/Gd, and may be used in the present invention. Preferred core materials are Au and Fe, with the most preferred material being Au. The cores of the nanoparticles preferably comprise between about 100 and 500 atoms (e.g. gold atoms) to provide core diameters in the nanometre range. Other particularly useful core materials are doped with one or more atoms that are NMR active, allowing the nanoparticles to be detected using NMR, both *in vitro* and *in vivo.* Examples of NMR active atoms include Mn⁺², Gd⁺³, Eu⁺², Cu⁺², V⁺², Co⁺², Ni⁺², Fe⁺², Fe⁺³ and lanthanides⁺³, or the quantum dots described elsewhere in this application.

Nanoparticle cores comprising semiconductor compounds can be detected as nanometre scale semiconductor crystals are capable of acting as quantum dots, that is they can absorb light thereby exciting electrons in the materials to higher energy levels, subsequently releasing photons of light at frequencies characteristic of the material. An example of a semiconductor core material is cadmium selenide, cadmium sulphide, cadmium tellurium. Also included are the zinc compounds such as zinc sulphide.

In some embodiments, the nanoparticle or its ligand comprises a detectable label. The label may be an element of the core of the nanoparticle or the ligand. The label may be detectable because of an intrinsic property of that element of the nanoparticle or by being linked, conjugated or associated with a further moiety that is detectable. Preferred examples of labels include a label which is a fluorescent group, a radionuclide, a magnetic label or a dye. Fluorescent groups include fluorescein, rhodamine or tetramethyl rhodamine, Texas-Red, Cy3, Cy5, etc., and may be detected by excitation of the fluorescent label and detection of the emitted light using Raman scattering spectroscopy (Y.C. Cao, R. Jin, C. A. Mirkin, Science 2002, 297: 1536-1539). In some cases, the detectable label may comprise fluorescein isothiocyanate (FITC). In certain cases, the detectable label (e.g. FITC) may be covalently linked to the core of the nanoparticle, e.g. via a linker.

In some embodiments, the nanoparticles may comprise a radionuclide for use in detecting the nanoparticle using the radioactivity emitted by the radionuclide, e.g. by using PET, SPECT, or for therapy, i.e. for killing target cells. Examples of radionuclides commonly used in the art that could be readily adapted for use in the present invention include ^{99m}Tc, which exists in a variety of oxidation states although the most stable is TcO⁴⁻; ³²P or ³³P; ⁵⁷Co; ⁵⁹Fe; ⁶⁷Cu which is often used as Cu²⁺ salts; ⁶⁷Ga which is commonly used a Ga³⁺ salt, e.g. gallium citrate; ⁶⁸Ge; ⁸²Sr; ⁹⁹Mo; ¹⁰³Pd; ¹¹¹In which is generally used as In³⁺ salts; ¹²⁵I or ¹³¹I which is generally used as sodium iodide; ¹³⁷Cs; ¹⁵³Gd; ¹⁵³Sm; ¹⁵⁸Au; ¹⁸⁶Re; ²⁰¹Tl generally used as a Tl⁺ salt such as thallium chloride; ³⁹Y³⁺; ⁷¹Lu³⁺; and ²⁴Cr²⁺. The general use of radionuclides as labels and tracers is well known in the art and could readily be adapted by the skilled person for use in the aspects of the present invention. The radionuclides may be employed most easily by doping the cores of the nanoparticles or including them as labels present as part of ligands immobilised on the nanoparticles.

### Actives

As used herein the term "biologically active agent" is intended to encompass drugs and pro-drugs that exert an effect on a biological system, preferably a therapeutic effect. Class of active agent contemplated herein include small molecule organic compounds, peptides, polypeptides and nucleic acids. Particular examples include: chemotherapeutic agents (e.g. temozolomide, irinotecan, chlorotoxin, carmustine, platinum(IV), platinum(II), camptothecin, doxorubicin, docetaxel Maytansine, Maytansinoids, monomethyl auristatin E (MMAE) and/or histone deacetylase (HDAC) inhibitors such as Panobinostat, Vorinostat, Romidepsin and Chidamide); peptides or polypeptides (e.g. insulin, GLP-1, amylin, exenatide, octreotide, teriparatide, glucagon, a cytokine, and/or an antibody); DNA or RNA (including, e.g. siRNA).

### Microparticles

Microparticles in accordance with the present invention may be in the form of solid beads. As used herein in connection with microparticles or beads, solid is intended to encompass a gel. Microparticle in accordance with the present invention has a diameter along its longest dimension that is within the range 10 micrometres to 75 micrometres. Microparticles contemplated herein advantageously include the monodisperse polymeric beads obtainable by the process described in WO 2012/042274.

### Process for encapsulating nanoparticles within microparticles

In certain cases the microparticles are produced by the Q Sphera® process as described in WO2012/042274.

### Administration and treatment

The microparticles and compositions of the invention may be administered to patients by any number of different routes, including enteral or parenteral routes. Parenteral administration includes administration by the following routes: intravenous, cutaneous or subcutaneous, nasal, intramuscular, intraocular, transepithelial, intraperitoneal and topical (including dermal, ocular, rectal, nasal, inhalation and aerosol), and rectal systemic routes.

Administration be performed e.g. by injection, especially depot injection.

The microparticles of the invention may be formulated as pharmaceutical compositions that may be in the forms of solid or liquid compositions. Such compositions will generally comprise a carrier of some sort, for example a solid carrier or a liquid carrier such as water, petroleum, animal or vegetable oils, mineral oil or synthetic oil. Physiological saline solution, or glycols such as ethylene glycol, propylene glycol or polyethylene glycol may be included. Such compositions and preparations generally contain at least 0.1 wt% of the compound.

For intravenous, cutaneous or subcutaneous injection, or injection at the site of affliction, the active ingredient will be in the form of a parenterally acceptable aqueous solution or liquid which is pyrogen-free and has suitable pH, isotonicity and stability. Those of relevant skill in the art are well able to prepare suitable solutions using, for example, solutions of the compounds or a derivative thereof, e.g. in physiological saline, a dispersion prepared with glycerol, liquid polyethylene glycol or oils.

In addition to one or more of the compounds, optionally in combination with other active ingredient, the compositions can comprise one or more of a pharmaceutically acceptable excipient, carrier, buffer, stabiliser, isotonicising agent, preservative or anti-oxidant or other materials well known to those skilled in the art. Such materials should be non-toxic and should not interfere with the efficacy of the active ingredient. The precise nature of the carrier or other material may depend on the route of administration, e.g., intramuscular injection.

Preferably, the pharmaceutically compositions are given to an individual in a prophylactically effective amount or a therapeutically effective amount (as the case may be, although prophylaxis may be considered therapy), this being sufficient to show benefit to the individual. Typically, this will be to cause a therapeutically useful activity providing benefit to the individual. The actual amount of the compounds administered, and rate and time-course of administration, will depend on the nature and severity of the condition being treated. Prescription of treatment, e.g. decisions on dosage etc., is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Handbook of Pharmaceutical Additives, 2nd Edition (eds. M. Ash and I. Ash), 2001 (Synapse Information Resources, Inc., Endicott, New York, USA); Remington's Pharmaceutical Sciences, 20th Edition, 2000, pub. Lippincott, Williams & Wilkins; and Handbook of Pharmaceutical Excipients, 2nd edition, 1994. By way of example, and the compositions are preferably administered to patients in dosages of between about 0.01 and 100mg of active compound per kg of body weight, and more preferably between about 0.5 and 10mg/kg of body weight.

The following is presented by way of example and is not to be construed as a limitation to the scope of the claims.

### Examples

### Example 1 - Synthesis of nanoparticles

Gold nanoparticles having a corona of carbohydrate ligands or glutathione ligands were synthesised essentially as described previously (WO 2011/154711; and Lund et al., 2011, Biomaterials Vol. 32 pp. 9776-9784).

### AL/α-Gal NPs

### Preparation of 2-thio-ethyl-α-D-galactoside (α-galactose C2SH)

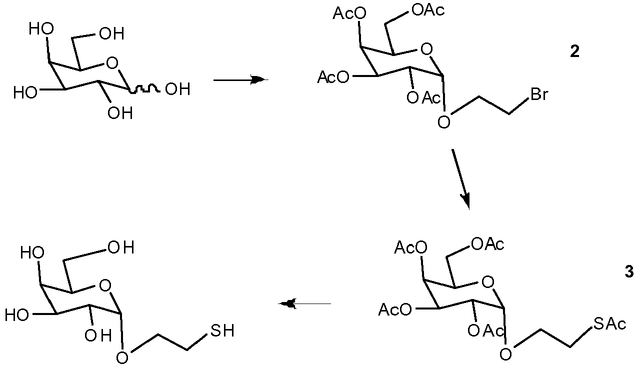

To a suspension of galactose (3g, 16.65 mmol) in 2-bromoethanol (30 ml), acid resin Amberlite 120-H is added to reach pH 2. The reaction is stirred for 16 hours at 50-60 ° C. The reaction mixture is filtered and washed with MeOH. Triethylamine is added to reach pH 8. The crude of the reaction is concentrated and co evaporated 3 times with toluene. The reaction mixture is dissolved pyridine (75 mL) and Ac20 (35 mL) and a catalytic amount of DMAP are added at 0°C and stirred for 3h at rt. The mixture is diluted with AcOEt and washed with 1.H₂O; 2.HCl (10%) 3. NaHCO₃ dis 4. H₂O. The organic layer is collected and dried over anhydrous Na₂SO₄. TLC (Hexane: AcOEt 3:1, 2 elutions) shows a major product (desired) and a lower Rf minority. The product is purified by flash chromatography using the mixture hexane: ethyl acetate 6:1 as eluent and the 2-bromoethyl-alpha-galactoside **(2)** is obtained.

The product of the previous reaction, **2** is dissolved in 27 ml of 2-butanone. To this solution, a catalytic amount of tetrabutylammonium iodide and 4 equivalents of potassium thioacetate are added. The resulting suspension is stirred for 2 hours at room temperature. Throughout this period the reaction is tested by TLC (hexane-AcOEt 2:1, 2 elutions) for the disappearance of the starting material. The mixture is diluted with 20ml of AcOEt and washed with a saturated NaCl solution. The organic phase is dried, filtered and evaporated under vacuum. The product is purified in hexane / AcOEt 2:1 → 1:1 to obtain the acetylthio-alpha-galactoside **3.**

The new product of the reaction, **3** is dissolved in a mixture dichloromethane-methanol 2:1. To this mixture a solution of 1N sodium methoxide (1 equivalent) is added and stirred for 1 hour at room temperature. Amberlite IR-120H resin is added to achieve pH 5-6. The resulting mixture is then filtered and concentrated to dryness to obtain the final product (a-galactose C2SH).

### Preparation of Amino-thiol linker.

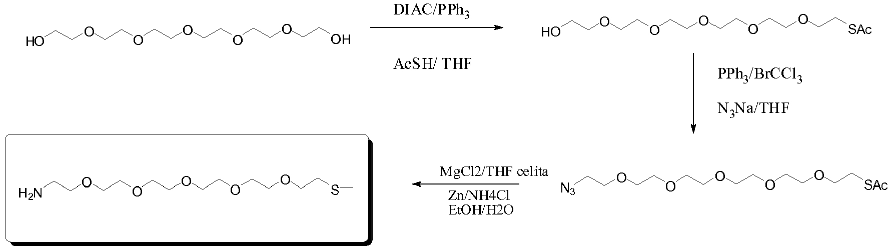

To a solution of PPh₃ (3g, 11.4 mmol) in 20 ml dry THF, DIAC (2.3g, 11.4mmol) is added. The mixture is allowed to stir at 0°C 15 min until the appearance of a white product. To this mixture a solution of hexaethyleneglycol (1.45mL, 5.7 mmol) and HSAc (610 µl, 8.55mmol) in dry THF (20 mL) is added dropwise (addition funnel). After 15 min the products begin to appear on TLC at Rf 0.2. The solution is concentrated in an evaporator. The crude of the reaction is dissolved in 50ml of dichloromethane and washed with a solution of K₂CO₃ 10%. The organic phase is dried over anhydrous Na₂SO₄, filtered and concentrated under vacuum. Flash chromatography of the crude using AcOEt: Hexane 1:1, AcOEt and finally DCM:MeOH 4:1 as eluent gave the acetyl-thio-hexaethyleneglycol derivative.

The reaction product is dissolved in 5 ml of DMF and PPh₃ (2.25g, 8.55mmol), NaN₃ (0.741g, 11.4mmol) and BrCl₃C (0,845 ml, 8.55mmol) are added and the solution subsequently stirred for 40 min at room temperature. The resulting product has a higher Rf than the starting product when performing TLC (DCM:MeOH 25:1). The reaction mixture is diluted with 100 ml of diethylether and washed three times with H₂O. The organic phase is dried over anhydrous Na₂SO₄, filtered and evaporated under vacuum. The product is purified by flash chromatography using the mixture of eluents DMC / MeOH 200:1 and DCM / MeOH 40:1 to obtain the azido-acetylthio-hexaethyleneglycol derivative.

To remove the triphenyl phosphine oxide, the reaction product is dissolved in 10 ml of THF and 0.5g of MgCl₂ is added to this solution. The reaction is stirred for 2h at 80°C until a white precipitate appears and then is filtered through celite.
The product is dissolved in a mixture of ethanol:H₂0 3:1 and added Zn dust (0.45g, 6.84mmol) and NH₄Cl (0.6g, 11.4mmol). The reaction was stirred at reflux for 1h until the presence of starting material is no longer detectable by TLC (DCM / MeOH 25:1). The reaction is filtered through celite and the solvent is evaporated. The crude de reaction is diluted with AcOEt and extract with 5 ml H₂0. The aqueous phase is evaporated to dryness to obtain the amino-thiol-hexaethylenglycol product.

Alpha-galactose C2 derivative **3** and hexaethyleneglycol amine linker **6** were taken from Midatech Biogune stock. *N*-(3-Dimethylaminopropyl)-*N*'-ethylcarbodiimide hydrochloride (EDC·HCl), HAuCl₄, NaBH₄ were purchased from Sigma-Aldrich Chemical Company. Imidazole-4-acetic acid monohydrochloride was purchased from Alfa Aesar. Company High quality MeOH and Nanopure water (18.1 mQ) were used for all experiments and solutions.

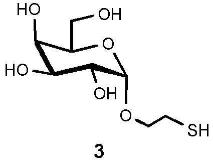

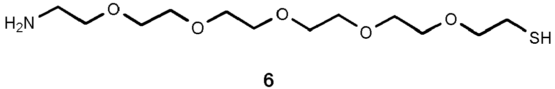

### α-GalC2 (alpha)

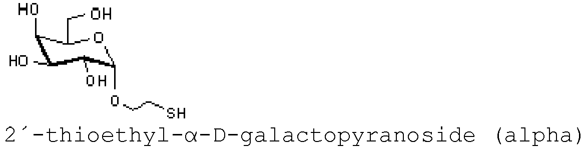

### EG6NH2

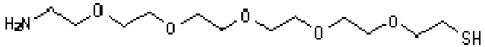

1-amino-17-mercapto-3,6,9,12,15,-pentaoxa-heptadecanol or 1-amino-6-mercapto-hexaethylenglycol (vulgar name)

Preparation of AL/α-Gal NPs: To a mix of amine-mercapto hexaethylenglycol linker **6** and alpha-galactose ligand **3** in a ratio 1:1 (0.58 mmol, 3 eq.) in MeOH (49 mL) was added an aqueous solution of gold salt (7.86 mL, 0.19 mmol, 0.025M). The reaction was stirred for 30 seconds and then, an aqueous solution of NaBH₄ (1N) was added in several portions (4.32 mL, 4.32 mmol). The reaction was shaken for 100 minutes at 900 rpm. After this time, the suspension was centrifuged 1 minute at 14000 rpm. The supernatant is removed and the precipitated was dissolved in 2 mL of water. Then, 2 mL of the suspension were introduced in two filters (AMICON, 10 KDa, 4 mL) and were centrifuged 5 minutes at 4500g. The residue in the filter was washed twice more with water. The final residue was dissolved in 80 mL of water.

For the preparation of gold NPs manufacture was under laminar flow cabinet. All glass and plastic material (such as eppendorfs, vials and bottles) and solvent (water, HAc) were first sterilized in an autoclave. All other disposables (such as tips and filters) came pre-sterilized.

### GSH NPs

Oxidized ligand, glutathione (Fluka 49741) was dissolved in 9:1 methanol:water and gold III chloride (Sigma-Aldrich, Poole, UK) added. The organic ligand was used at a fourfold molar excess relative to the gold. The solution was then mixed for 5 min gently on a flat-bed shaker. The nanoparticles were produced by reduction following the rapid addition of a 20 fold molar excess relative to the gold, of freshly made 1 M sodium borohydride (Sigma-Aldrich, Poole, UK) under vigorous vortexing. The samples were vortexed for a total of 30 s followed by a further 1 h gentle mixing on the flat bed shaker. As the nanoparticles are not soluble in methanol/water solvent, initial purification was by bench centrifugation, supernatant removal and dispersion of the nanoparticle pellet in water. Further purification was achieved by 4 water washes in 10 kDa vivaspin centrifugation devices (GE Healthcare). The gold concentration of all nanoparticle preparations was determined by a simple colorimetric assay. Briefly 10 µl of nanoparticle sample or 12 mg/ml gold standard (Fluka (Sigma-Aldrich, Poole, UK)) and blanks were digested with 30 µl of 50:50 water:aqua regia in an ELISA plate for 1 min, this was followed by addition of 150 µl of 2 M NaBr, the 405 nm absorbance was then measured immediately, the assay having excellent linearity over the 0-10 µg range.

### Colloidal gold nanoparticles

Colloidal gold nanoparticles were prepared in-house using standard techniques.

### Example 2 - Encapsulation of nanoparticles within polymeric microparticles

Encapsulation of gold nanoparticles (GNPs) in PLGA microparticles (solid beads) was performed using the following three GNPs solutions:
- NP-PEGamine/aGal (i.e. GNPs having a corona of an approximately 50:50 ratio of PEGamine ligands (1-amino-17-mercapto-3,6,9,12,15,-pentaoxa-heptadecanol) to C2-alpha-galactose ligands (2-thio-ethyl-α-D-galactoside)) dissolved in a mixture of 90:10 DMSO/Water (1mg/mL) ["**NP1**"].
- NP-Glutathione (i.e. GNPs having a corona of glutathione ligands) dissolved in a mixture of 90:10 DMSO/Water (1mg/mL) ["**NP2**"].
- Colloidal gold in a mixture of 90:10 DMSO/Water (0.18mg/mL) ["**NP3**"].

As PLGA is insoluble in water, it is difficult to produce a stable solution of PLGA, DMSO and water. As a maximum, a mixture containing up to ∼10% water can be generated.

The present inventors therefore sought to obtain gold nanoparticles (GNPs) prepared in a DMSO - water mixed phase. Highly concentrated solution of GNPs are contemplated for use herein.

To prepare a solution of PLGA and GNPs, first a highly concentrated solution of PLGA in DMSO (60-80% w/v) was prepared. This was a linear PLGA (L/G 75:25) with a carboxylic acid terminus, molecular weight ∼ 10kDaltons. This solution was then diluted by 33%-50% with one of the above GNP solutions, to yield a final polymer concentration of 40% w/v.

A solution of antisolvent (85:15 Water/tert-Butanol) was produced and cooled to ∼4°C. The solution was then placed in a piezoelectric droplet generating device (see WO2012/042273) and a stable droplet stream was produced. Droplets were allowed to fall into and react with the above antisolvent solution at a temperature of between 5-8°C. Rapid extraction reaction took place, producing solid microparticles of diameter ∼53µm.
The solid microspheres were dried *in vacuo* using a Bead Harvester device (see WO2013/014466) and stored at 4°C.

### Characterisation of nanoparticle-encapsulating microparticles

In order to quantify the encapsulation efficiency, a spectrophotometric assay was undertaken using NP3 (which has a visible absorption at ∼520nm). A serial dilution was prepared in acetonitrile. An accurate mass of GNP-containing microspheres was then dissolved in acetonitrile and the absorption of the solution quantified. Using this method, we determined that NP3 was successfully encapsulated with an entrapment efficiency of ∼96%.

Using UV-Vis spectrophotometry, we also determined that encapsulated samples of NP1 and NP2 had not aggregated during the process, i.e. no UV-Vis absorption bands were detected after dissolution of microspheres in acetonitrile. The present inventors contemplate loading & release studies making use of inductively coupled plasma mass spectrometry (ICPMS) techniques for quantification.

A quantity (3.5 mg) of PLGA microparticles encapsulating NP2 (i.e. glutathione nanoparticles) was treated with 200 µl 50% *aqua regia* and the microparticles solubilised visually to >50% (some particles could still be seen). The material was centrifuged and the supernatant tested for gold content using an in-house colorimetric assay.

30 µl supernatant gave 0.21 µg Au, which equates to 1.4 µg Au/3.5mg PLGA-NP microparticle. Based on a theoretical maximum of 1.94 µg gold of NP2 if encapsulation efficiency had been 100%, these results represent 72% recovery. The present inventors conclude that a high level of nanoparticle incorporation into PLGA microspheres was demonstrated.

### Example 3 - Encapsulation of gold nanoparticles within PLGA microspheres

Further examples of nanoparticles have been encapsulated and characterised essentially as described in Example 2.

### Experiment 1

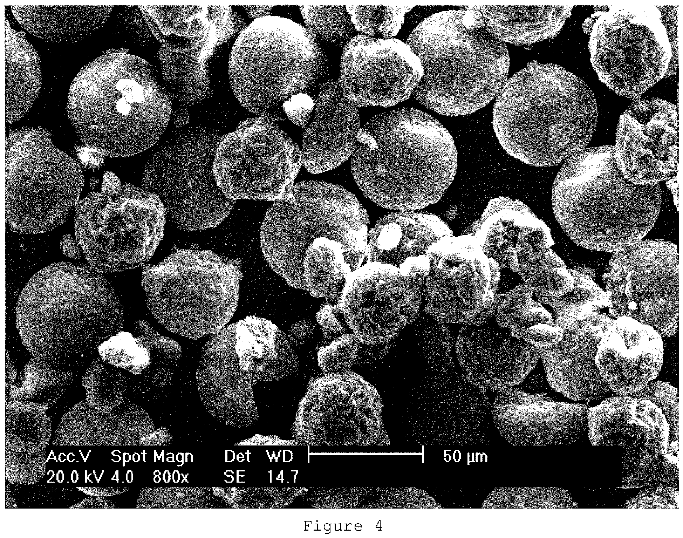

40% w/v RG752H (Resomer® RG 752 H, Poly(D,L-lactide-co-glycolide) acid terminated, lactide:glycolide 75:25, Mw 4,000-15,000) in DMSO with 0.25mg/mL C2-glucose GNPs (9:1 DMSO:Water) successfully produced microspheres using a piezoelectric droplet generator device. The antisolvent was 15% v/v tertiary butanol at room temperature. An electron micrograph showing the microparticles can be seen in Figure 4.

The average diameter of the microparticles was **53.49 µm;** Standard deviation **4.34 µm;** CV **8.12%.**

### Experiment 2

40% w/v RG752H in DMSO with 0.5mg/mL C2-glucose GNPs (9:1 DMSO:Water) successfully produced microspheres using the piezoelectric droplet generator device. Antisolvent - 15% v/v tertiary butanol at room temperature.

Average diameter - **46.67µm** Standard deviation - **2.11µm** CV - **4.53** %.

### Experiment 3

40% w/v RG752H in DMSO with 0.18mg/mL Colloidal Gold (9:1 DMSO:Water) successfully produced microspheres using the piezoelectric droplet generator device. Antisolvent - 15% v/v tertiary butanol at room temperature.

Average diameter - **44.82pm** Standard deviation - **3.35µm** CV - **7.47%.**

As colloid gold is detectable by UV spectrophotometry at 520 nm the concentration within the microspheres was analysed. It produced a loading of 0.174 mg/mL which produced an encapsulation efficiency of 96.4%.

### Experiment 4

### Encapsulation of FITC-conjugated GNPs

FITC-PEG-SH-1kda GNPs c(Au) 0.587g/l were centrifuged and resuspended in 250µL DMSO. Transferred to 33% w/v RG752H in DMSO formulation. Successfully manufactured with the piezoelectric droplet generator using 15% v/v tertiary butanol as the antisolvent.

Average diameter - **59.4pm** Standard deviation - **3µm** CV - **5.1%**

In 1mg of GNP microspheres there were 2.18µg/mL of the FITC-PEG-SH-1kda detected.

The successful encapsulation of nanoparticles coupled to a detectable label illustrates the provision of encapsulated, payload-coupled nanoparticles according to the present invention.

### Experiment 5

### Encapsulation of glutathione-conjugated GNPs

Glutathione-conjugated GNPs c(Au) 6.52mg/mL were centrifuged for 30 minutes in amicon to remove the majority of water then the GNPs were resuspended in 200 µL of DMSO. 200µL was then added to a 30% w/v RG752H formulation in DMSO. This was then used to manufacture microspheres using the piezoelectric droplet generator. 15% v/v tertiary butanol was used as the antisolvent.

Average diameter - **49.36µm** Standard deviation - **4.48pm** CV - **9.07%**

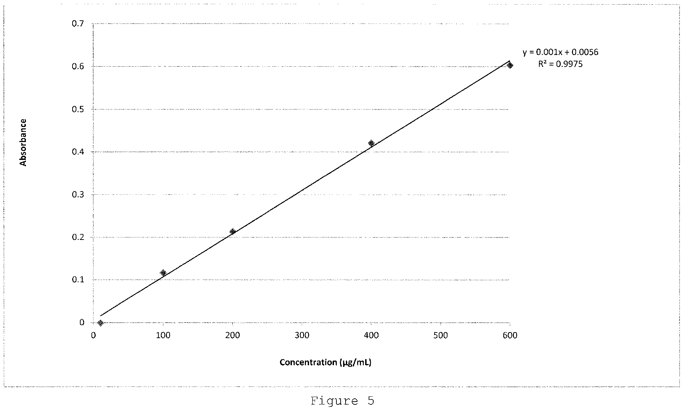

Nanoparticles were dissolved in aqua regia and a colorimetric assay used to measure gold concentration. The assay was used to produce a standard curve using the gold AAS standard solution read at 382 nm. A linear trend was shown between a HLQ of 600 µg/mL and an LLQ of 10 µg/mL (see Figure 5).

The microspheres produced a gold concentration of 0.8279 mg/mL. The formulation theoretical maximum - 1.304mg/mL gold concentration, which gives an encapsulation efficiency of 63.49%.

The specific embodiments described herein are offered by way of example, not by way of limitation. Any sub-titles herein are included for convenience only, and are not to be construed as limiting the disclosure in any way.

## Claims

1. A microparticle comprising at least one biocompatible polymer, the microparticle encapsulating at least one nanoparticle, the nanoparticle comprising:
(i) a core comprising a metal and/or a semiconductor;
(ii) a corona comprising a plurality of ligands covalently linked to the core, wherein said ligands comprise at least one carbohydrate and/or glutathione,
wherein the microparticle has a diameter along its longest dimension that is within the range 10 µm to 75 µm.

2. The microparticle according to claim 1, wherein the at least one nanoparticle further comprises at least one biologically active agent or detectable label covalently linked or non-covalently bound to said corona and/or said core.

3. The microparticle according to claim 2, wherein:
(i) the at least one biologically active agent comprises a chemotherapeutic agent and is covalently linked to said core via a linker;
(ii) the at least one biologically active agent is covalently linked to said core via a linker comprising C2-C15 alkyl and/or C2-C15 glycol; and/or
(iii) the at least one biologically active agent is a chemotherapeutic agent selected from the group consisting of: temozolomide, irinotecan, chlorotoxin, carmustine, platinum(IV), platinum(II), camptothecin, doxorubicin, docetaxel, maytansine, a maytansinoids, monomethyl auristatin E (MMAE), and a histone deacetylase (HDAC) inhibitor.

4. The microparticle according to any one of the preceding claims, wherein the nanoparticle further comprises a cell, tissue or tumour targeting moiety, optionally wherein said targeting moiety is selected from the group consisting of: folic acid, lactose, albumin, glutamine, a ligand that binds a cell surface receptor, and an antibody.

5. The microparticle according to any one of the preceding claims, wherein:
(i) the diameter of the core of the nanoparticle is in the range 1 nm to 5 nm;
(ii) the diameter of the nanoparticle including its ligands is in the range 2 nm to 50 nm, or 3 nm to 30 nm, or 4 nm to 20 nm, or 5 nm to 15 nm; and/or
(iii) the core comprises a metal selected from the group consisting of: Au, Ag, Cu, Pt, Pd, Fe, Co, Gd, Eu, and Zn, or any combination thereof.

6. The microparticle according to any one claims 2 to 5, wherein the microparticle comprises a plurality of said nanoparticles, wherein at least 10%, 20%, 30%, 40%, 50%, 60%, 70%, 80%, 90%, 95% or 99% of the nanoparticles in said microparticle have at least one biologically active agent conjugated or bound.

7. The microparticle according to any one claims 2 to 6, wherein:
(i) the microparticle comprises at least two different species of biologically active agent;
(ii) the microparticle comprises at least two different species of biologically active agent, wherein a first of said at least two different species of biologically active agent is conjugated or bound to a first nanoparticle and a second of said at least two different species of biologically active agent is conjugated or bound to a second nanoparticle;
(iii) the microparticle comprises at least two different species of biologically active agent, wherein said at least one nanoparticle has at least two different species of biologically active agent conjugated or bound thereto; and/or
(iv) the average density of biologically active agent expressed in terms of % weight of agent per weight of microparticle polymer is within the range 0.005% to 20% w/w, optionally in the range 0.01% to 10% w/w.

8. The microparticle according to any one of the preceding claims, wherein:
(i) the microparticle has a diameter along its longest dimension that is within the range 25 µm to 75 µm;
(ii) the morphology of the microparticle is substantially spherical or is lenticular;
(iii) the surface porosity of the microparticle is **characterised by** an average surface pore size of <20 nm, <100 nm, < 1 µm or < 10 µm;
(iv) said at least one biocompatible polymer is selected from the group consisting of: a polylactide, a polycaprolactone, a polyanhydride and a co-polymer of lactic acid and glycolic acid ("PLGA"), optionally wherein:
(a) the said polymer or co-polymer comprises at least one end group or terminal functional group selected from the group consisting of: carboxyl, hydroxyl and ester;
(b) the polymer or co-polymer comprises at least one end group or terminal functional group that binds to a functional group present on the corona of said nanoparticle;
(v) the average concentration of nanoparticles expressed in terms of % weight of nanoparticle per weight of microparticle polymer is within the range 0.01% to 25% w/w, optionally in the range 0.05% to 10% w/w; and/or
(vi) the *in vivo* release profile of the microparticle following depot injection of the microparticle in a mammalian subject is in the range 1 week to 6 months, optionally in the range 3 weeks to 3 months.

9. A pharmaceutical composition comprising a plurality of microparticles as defined in any one of the preceding claims and a pharmaceutically acceptable carrier or excipient, optionally wherein:
(i) the composition comprises an anti-agglomerating agent;
(ii) the carrier is an aqueous or organic liquid, such as a biocompatible oil; and/or
(iii) the composition is formulated for delivery via an injectable route, such as subcutaneous or intramuscular depot injection, or formulated for implantation during surgery.

10. A microparticle as defined in any one of claims 1 to 8 or a pharmaceutical composition according to claim 9 for use in medicine, wherein said at least one nanoparticle further comprises at least one biologically active agent comprising a drug or pro-drug that exerts a therapeutic effect.

11. A microparticle as defined in any one of claims 1 to 8 or a pharmaceutical composition according to claim 9 for use in a method of treatment of a cancer in a mammalian subject, wherein said microparticle comprises a chemotherapeutic agent.

12. A microparticle or pharmaceutical composition for use according to claim 11, wherein:
(i) the cancer is selected from the group consisting of: a brain cancer, optionally glioblastoma, glioma or astrocytoma; an ovarian cancer; a liver cancer; and a skin cancer, and wherein said chemotherapeutic agent is selected from the group consisting of: temozolomide, irinotecan, chlorotoxin, carmustine, platinum(IV), platinum(II), camptothecin, doxorubicin, docetaxel, maytansine, a maytansinoid, monomethyl auristatin E (MMAE) and a histone deacetylase (HDAC) inhibitor; or
(ii) the time interval between administration of doses is at least 1 week, optionally at least 2, 3, or 4 weeks, and further optionally at least 1, 2 or 3 months.

13. An article of manufacture comprising:
microparticle as defined in any one of claims 1 to 8 or a pharmaceutical composition according to claim 9;
a container for housing the microparticle or pharmaceutical composition; and
an insert and/or label.

14. A process for producing a microparticle as defined in any one of claims 1 to 8, the process comprising:
providing a first liquid comprising a solute, a solvent and a plurality of nanoparticles intended to be encapsulated within the microparticles, the solute comprising a polymer, the concentration of polymer in the first liquid being at least 10% w/v, 'w' being the weight of the polymer and 'v' being the volume of the solvent, the nanoparticles comprising:
(i) a core comprising a metal and/or a semiconductor; and (ii) a corona comprising a plurality of ligands covalently linked to the core, wherein said ligands comprise at least one carbohydrate and/or glutathione;
providing a liquid droplet generator comprising a piezoelectric component operable to generate liquid droplets,
causing the liquid droplet generator to form liquid droplets of the first liquid;
passing the liquid droplets through a gas,
contacting the liquid droplets with a second liquid so as to cause the solvent to exit the droplets, thus forming solid microparticles;
the solubility of the solvent in the second liquid being at least 5g of solvent per 100ml of second liquid, the solvent being substantially miscible with the second liquid,
wherein the second liquid is provided as a flow and the method comprises contacting the liquid droplets with the flow of second liquid.

15. The process according to claim 14, wherein:
(i) the nanoparticles are provided in an organic solvent:water mixed phase; and/or
(ii) the process further comprises collecting the solid microparticles and formulating or packaging the microparticles into a pharmaceutical composition or delivery form.

## Patentansprüche

1. Mikroteilchen, umfassend zumindest ein biokompatibles Polymer, wobei das Mikroteilchen zumindest ein Nanoteilchen einkapselt, wobei das Nanoteilchen Folgendes umfasst:
(i) einen Kern, umfassend ein Metall und/oder einen Halbleiter;
(ii) eine Corona, umfassend eine Vielzahl von Liganden, die kovalent an den Kern gebunden sind, wobei die Liganden zumindest ein Kohlenhydrat und/oder Glutathion umfassen,
wobei das Mikroteilchen einen Durchmesser entlang der längsten Abmessung aufweist, der im Bereich von 10 µm bis 75 µm liegt.

2. Mikroteilchen nach Anspruch 1, wobei das zumindest eine Nanoteilchen weiters zumindest ein biologisch aktives Mittel oder eine detektierbare Markierung umfasst, das/die kovalent oder nicht kovalent an die Corona und/oder den Kern gebunden ist.

3. Mikroteilchen nach Anspruch 2, wobei:
(i) das zumindest eine biologisch aktive Mittel ein Chemotherapeutikum umfasst und über einen Linker kovalent an den Kern gebunden ist;
(ii) das zumindest eine biologisch aktive Mittel über einen Linker, umfassend C₂₋₁₅-Alkyl und/oder C₂₋₁₅-Glykol, kovalent an den Kern gebunden ist; und/oder
(iii) das zumindest eine biologisch aktive Mittel ein Chemotherapeutikum ist, das aus der aus folgenden bestehenden Gruppe ausgewählt ist: Temozolomid, Irinotecan, Chlorotoxin, Carmustin, Platin(IV), Platin(II), Camptothecin, Doxorubicin, Docetaxel, Maytansin, einem Maytansinoid, Monomethylauristatin E (MMAE) und einem Histondeacetylase-(HDAC-) Inhibitor.

4. Mikroteilchen nach einem der vorangegangenen Ansprüche, wobei das Nanoteilchen weiters eine auf eine Zelle, ein Gewebe oder eine auf Tumor abzielende Gruppierung umfasst, gegebenenfalls wobei die abzielende Gruppierung aus der aus folgenden bestehenden Gruppe ausgewählt ist: Folsäure, Lactose, Albumin, Glutamin, einem Liganden, der an einen Zelloberflächenrezeptor bindet, und einem Antikörper.

5. Mikroteilchen nach einem der vorangegangenen Ansprüche, wobei:
(i) der Durchmesser des Kerns des Nanoteilchens im Bereich von 1 nm bis 5 nm liegt;
(ii) der Durchmesser des Nanoteilchens, einschließlich seiner Liganden, im Bereich von 2 nm bis 50 nm oder 3 nm bis 30 nm oder 4 nm bis 20 nm oder 5 nm bis 15 nm liegt; und/oder
(iii) der Kern ein Metall umfasst, das aus der aus folgenden bestehenden Gruppe ausgewählt ist: Au, Ag, Cu, Pt, Pd, Fe, Co, Gd, Eu und Zn oder einer beliebigen Kombination daraus.

6. Mikroteilchen nach einem der Ansprüche 2 bis 5, wobei das Mikroteilchen eine Vielzahl der Nanoteilchen umfasst, wobei zumindest 10 %, 20 %, 30 %, 40 %, 50 %, 60 %, 70 %, 80 %, 90 %, 95 % oder 99 % der Nanoteilchen in dem Mikroteilchen zumindest ein daran konjugiertes oder gebundenes biologisch aktives Mittel aufweisen.

7. Mikroteilchen nach einem der Ansprüche 2 bis 6, wobei:
(i) das Mikroteilchen zumindest zwei verschiedene Spezies eines biologisch aktiven Mittels umfasst;
(ii) das Mikroteilchen zumindest zwei verschiedene Spezies eines biologisch aktiven Mittels umfasst, wobei eine erste der zumindest zwei unterschiedlichen Spezies eines biologisch aktiven Mittels an ein erstes Nanoteilchen konjugiert oder gebunden ist und eine zweite aus den zumindest zwei verschiedenen Spezies eines biologisch aktiven Mittels an ein zweites Nanoteilchen konjugiert oder gebunden ist;
(iii) das Mikroteilchen zumindest zwei verschiedene Spezies eines biologisch aktiven Mittels umfasst, wobei das zumindest eine Nanoteilchen zumindest zwei verschiedene Spezies eines biologisch wirksamen Mittels aufweist, die daran konjugiert oder gebunden sind; und/oder
(iv) die durchschnittliche Dichte des biologisch aktiven Mittels, die in Gew.-% des Mittels pro Gewicht des Mikroteilchen-Polymers ausgedrückt wird, im Bereich von 0,005 Gew.-% bis 20 Gew.-%, gegebenenfalls im Bereich von 0,01 Gew.-% bis 10 Gew.-%, liegt.

8. Mikroteilchen nach einem der vorangegangenen Ansprüche, wobei:
(i) das Mikroteilchen einen Durchmesser entlang der längsten Abmessung aufweist, der im Bereich von 25 µm bis 75 µm liegt;
(ii) die Morphologie des Nanoteilchens im Wesentlichen kugelförmig oder linsenförmig ist;
(iii) die Oberflächenporosität des Mikroteilchens durch eine durchschnittliche Oberflächenporengröße von < 20 nm, < 100 nm, < 1 µm oder < 10 µm gekennzeichnet ist;
(iv) das zumindest eine biokompatible Polymer aus der aus folgenden bestehenden Gruppe ausgewählt ist: einem Polylactid, einem Polycaprolacton, einem Polysäureanhydrid und einem Copolymer von Milchsäure und Glykolsäure ("PLGA"), gegebenenfalls wobei:
(a) das Polymer oder das Copolymer zumindest eine endständige Gruppe oder terminale funktionelle Gruppe umfasst, die aus der aus folgenden bestehenden Gruppe ausgewählt ist: Carboxyl, Hydroxyl und Ester;
(b) das Polymer oder Copolymer zumindest eine endständige Gruppe oder terminale funktionelle Gruppe umfasst, die an eine funktionelle Gruppe bindet, die auf der Corona des Nanoteilchens vorhanden ist;
(v) die durchschnittliche Konzentration von Nanoteilchen, die in Gew.-% des Nanoteilchens pro Gewicht des Mikroteilchenpolymers ausgedrückt wird, im Bereich von 0,01 Gew.-% bis 25 Gew.-%, gegebenenfalls im Bereich von 0,05 Gew.-% bis 10 Gew.-%, liegt; und/oder
(vi) das In-vivo-Freisetzungsprofil des Mikroteilchens nach einer Depot-Injektion des Mikroteilchens in ein Säugetierindividuum im Bereich von 1 Woche bis 6 Monaten, gegebenenfalls im Bereich von 3 Wochen bis 3 Monaten, liegt.

9. Pharmazeutische Zusammensetzung, umfassend eine Vielzahl von Mikroteilchen, wie in einem der vorangegangenen Ansprüche definiert, und einen pharmazeutisch annehmbaren Träger oder Exzipienten, gegebenenfalls wobei:
(i) die Zusammensetzung ein Anti-Agglomerationsmittel umfasst;
(ii) der Träger eine wässrige oder organische Flüssigkeit wie ein biokompatibles Öl ist; und/oder
(iii) die Zusammensetzung zur Anlieferung über eine Injektionsroute wie eine subkutane oder intramuskuläre Depot-Injektion formuliert ist oder zur Implantation während eines chirurgischen Eingriffs formuliert ist.

10. Mikroteilchen wie in einem der Ansprüche 1 bis 8 definiert oder pharmazeutische Zusammensetzung nach Anspruch 9 zur Verwendung auf dem Gebiet der Medizin, wobei das zumindest eine Nanoteilchen weiters zumindest ein biologisch aktives Mittel, umfassend ein Arzneimittel oder ein Prodrug, das eine therapeutische Wirkung aufweist, umfasst.

11. Mikroteilchen wie in einem der Ansprüche 1 bis 8 definiert oder pharmazeutische Zusammensetzung nach Anspruch 9 zur Verwendung in einem Verfahren zur Behandlung einer Krebserkrankung in einem Säugetierindividuum, wobei das Mikroteilchen ein Chemotherapeutikum umfasst.

12. Mikroteilchen oder pharmazeutische Zusammensetzung zur Verwendung nach Anspruch 11, wobei:
(i) die Krebserkrankung aus der aus folgenden bestehenden Gruppe ausgewählt ist: einem Hirnkrebs, gegebenenfalls Glioblastom, Gliom oder Astrozytom; einem Eierstocckrebs; einem Leberkrebs; und einem Hautkrebs, und wobei das Chemotherapeutikum aus der aus folgenden bestehenden Gruppe ausgewählt ist: Temozolomid, Irinotecan, Chlorotoxin, Carmustin, Platin(IV), Platin(II), Camptothecin, Doxorubicin, Docetaxel, Maytansin, einem Maytansinoid, Monomethylauristatin E (MMAE) und einem Histondeacetylase-(HDAC-) Inhibitor; oder
(ii) der zeitliche Abstand zwischen der Verabreichung von Dosen zumindest 1 Woche, gegebenenfalls zumindest 2, 3 oder 4 Wochen und weiters gegebenenfalls zumindest 1, 2 oder 3 Monate beträgt.

13. Fabrikat, umfassend:
Mikroteilchen wie in einem der Ansprüche 1 bis 8 definiert oder eine pharmazeutische Zusammensetzung nach Anspruch 9;
einen Behälter zum Einhausen des Mikroteilchens oder der pharmazeutischen Zusammensetzung; und
eine Packungsbeilage und/oder ein Etikett.

14. Verfahren zur Herstellung eines Mikroteilchens wie in einem der Ansprüche 1 bis 8 definiert, wobei das Verfahren Folgendes umfasst:
Bereitstellen einer ersten Flüssigkeit, umfassend einen Gelöststoff, ein Lösungsmittel und eine Vielzahl von Nanoteilchen, die innerhalb der Mikroteilchen einzukapseln sind, wobei der Gelöststoff ein Polymer umfasst, wobei die Konzentration des Polymers in der ersten Flüssigkeit zumindest 10 % (Gew./Vol.) aufweist, wobei 'Gew.' das Gewicht des Polymers bezeichnet und 'Vol.' das Volumen des Lösungsmittels bezeichnet, wobei die Nanoteilchen Folgendes umfassen:
(i) einen Kern, umfassend ein Metall und/oder einen Halbleiter; und (ii) eine Corona, umfassend eine Vielzahl von Liganden, die kovalent an den Kern gebunden ist, wobei die Liganden zumindest ein Kohlenhydrat und/oder Glutathion umfassen;
Bereitstellen einer Flüssigkeitströpfchenerzeugungsvorrichtung, umfassend eine piezoelektrische Komponente, die betreibbar ist, um Flüssigkeitströpfchen zu erzeugen,
Bewirken, dass die Flüssigkeitströpfchenerzeugungsvorrichtung Flüssigkeitströpfchen der ersten Flüssigkeit bildet;
Durchströmen der Flüssigkeitströpfchen durch ein Gas,
Kontaktieren der Flüssigkeitströpfchen mit einer zweiten Flüssigkeit, um zu bewirken, dass das Lösungsmittel aus den Tröpfchen austritt, wodurch feste Mikroteilchen gebildet werden;
wobei die Löslichkeit des Lösungsmittels in der zweiten Flüssigkeit bei zumindest 5 g Lösungsmittel pro 100 ml der zweiten Flüssigkeit liegt, wobei das Lösungsmittel im Wesentlichen mit der zweiten Flüssigkeit mischbar ist,
wobei die zweite Flüssigkeit als eine Strömung bereitgestellt ist und das Verfahren das Kontaktieren der Flüssigkeitströpfchen mit der Strömung der zweiten Flüssigkeit umfasst.

15. Verfahren nach Anspruch 14, wobei:
(i) die Nanoteilchen in einer Mischphase aus organischem Lösungsmittel und Wasser bereitgestellt sind; und/oder
(ii) das Verfahren weiters das Gewinnen der festen Mikroteilchen und das Formulieren oder Verpacken der Mikroteilchen in eine pharmazeutische Zusammensetzung oder Anlieferungsform umfasst.

## Revendications

1. Microparticule comprenant au moins un polymère biocompatible, la microparticule encapsulant au moins une nanoparticule, la nanoparticule comprenant :
(i) un noyau comprenant un métal et/ou un semi-conducteur ;
(ii) une couronne comprenant une pluralité de ligands liés de façon covalente au noyau, où lesdits ligands comprennent au moins un glucide et/ou le glutathion,
où la microparticule a un diamètre le long de sa plus longue dimension qui est dans la plage de 10 µm à 75 µm.

2. Microparticule selon la revendication 1, dans laquelle l'au moins une nanoparticule comprend en outre au moins un agent biologiquement actif ou un marqueur détectable lié de façon covalente ou lié de façon non covalente à ladite couronne et/ou audit noyau.

3. Microparticule selon la revendication 2, dans laquelle :
(i) l'au moins un agent biologiquement actif comprend un agent chimiothérapeutique et est lié de façon covalente audit noyau par l'intermédiaire d'un lieur ;
(ii) l'au moins un agent biologiquement actif est lié de façon covalente audit noyau par l'intermédiaire d'un lieur comprenant alkyle en C2-C15 et/ou glycol en C2-C15 ; et/ou
(iii) l'au moins un agent biologiquement actif est un agent chimiothérapeutique choisi dans le groupe constitué des : témozolomide, irinotécan, chlorotoxine, carmustine, platine(IV), platine(II), camptothécine, doxorubicine, docétaxel, maytansine, un maytansinoïde, monométhyl-auristatine E (MMAE) et un inhibiteur d'histone désacétylase (HDAC).

4. Microparticule selon l'une quelconque des revendications précédentes, où la nanoparticule comprend en outre un fragment de ciblage cellulaire, tissulaire ou tumoral, facultativement où ledit fragment de ciblage est choisi dans le groupe constitué des : acide folique, lactose, albumine, glutamine, un ligand qui se lie à un récepteur de surface cellulaire, et un anticorps.

5. Microparticule selon l'une quelconque des revendications précédentes, dans laquelle :
(i) le diamètre du noyau de la nanoparticule est dans la plage de 1 nm à 5 nm ;
(ii) le diamètre de la nanoparticule comprenant ses ligands est dans la plage de 2 nm à 50 nm, ou de 3 nm à 30 nm, ou de 4 nm à 20 nm, ou de 5 nm à 15 nm ; et/ou
(iii) le noyau comprend un métal choisi dans le groupe constitué de : Au, Ag, Cu, Pt, Pd, Fe, Co, Gd, Eu et Zn, ou une combinaison quelconque de ceux-ci.

6. Microparticule selon l'une quelconque des revendications 2 à 5, où la microparticule comprend une pluralité desdites nanoparticules, où au moins 10 %, 20 %, 30 %, 40 %, 50 %, 60 %, 70 %, 80 %, 90 %, 95 % ou 99 % des nanoparticules dans ladite microparticule comportent au moins un agent biologiquement actif conjugué ou lié.

7. Microparticule selon l'une quelconque des revendications 2 à 6, où :
(i) la microparticule comprend au moins deux espèces différentes d'agent biologiquement actif ;
(ii) la microparticule comprend au moins deux espèces différentes d'agent biologiquement actif, où une première desdites au moins deux espèces différentes d'agent biologiquement actif est conjuguée ou liée à une première nanoparticule et une deuxième desdites au moins deux espèces différentes d'agent biologiquement actif est conjuguée ou liée à une deuxième nanoparticule ;
(iii) la microparticule comprend au moins deux espèces différentes d'agent biologiquement actif, où ladite au moins une nanoparticule comporte au moins deux espèces différentes d'agent biologiquement actif conjuguées ou liées à celle-ci ; et/ou
(iv) la densité moyenne d'agent biologiquement actif exprimée en termes de % en poids d'agent par poids de polymère de microparticule est dans la plage de 0,005 % à 20 % m/m, facultativement dans la plage de 0,01 % à 10 % m/m.

8. Microparticule selon l'une quelconque des revendications précédentes, où :
(i) la microparticule a un diamètre le long de sa plus grande dimension qui est dans la plage de 25 µm à 75 µm ;
(ii) la morphologie de la microparticule est sensiblement sphérique ou est lenticulaire ;
(iii) la porosité de surface de la microparticule est **caractérisée par** une taille de pore de surface moyenne de < 20 nm, < 100 nm, < 1 µm ou < 10 µm ;
(iv) ledit au moins un polymère biocompatible est choisi dans le groupe constitué de : un polylactide, une polycaprolactone, un polyanhydride et un copolymère d'acide lactique et d'acide glycolique (« PLGA »), facultativement dans laquelle :
(a) ledit polymère ou copolymère comprend au moins un groupe d'extrémité ou groupe fonctionnel terminal choisi dans le groupe constitué de : carboxyle, hydroxyle et ester ;
(b) le polymère ou copolymère comprend au moins un groupe d'extrémité ou groupe fonctionnel terminal qui se lie à un groupe fonctionnel présent sur la couronne de ladite nanoparticule ;
(v) la concentration moyenne de nanoparticules exprimée en termes de % en poids de nanoparticule par poids de polymère de microparticule est dans la plage de 0,01 % à 25 % m/m, facultativement dans la plage de 0,05 % à 10 % m/m ; et/ou
(vi) le profil de libération *in vivo* de la microparticule après injection retard de la microparticule chez un sujet mammifère est dans la plage de 1 semaine à 6 mois, facultativement dans la plage de 3 semaines à 3 mois.

9. Composition pharmaceutique comprenant une pluralité de microparticules telles que définies dans l'une quelconque des revendications précédentes et un véhicule ou excipient pharmaceutiquement acceptable, facultativement où :
(i) la composition comprend un agent antiagglomérant ;
(ii) le véhicule est un liquide aqueux ou organique, tel qu'une huile biocompatible ; et/ou
(iii) la composition est formulée pour administration par une voie injectable, telle qu'une injection retard sous-cutanée ou intramusculaire, ou formulée pour implantation pendant une chirurgie.

10. Microparticule telle que définie dans l'une quelconque des revendications 1 à 8 ou composition pharmaceutique selon la revendication 9, pour utilisation en médecine, dans laquelle ladite au moins une nanoparticule comprend en outre au moins un agent biologiquement actif comprenant un médicament ou un promédicament qui exerce un effet thérapeutique.

11. Microparticule telle que définie dans l'une quelconque des revendications 1 à 8 ou composition pharmaceutique selon la revendication 9, pour utilisation dans un procédé de traitement d'un cancer chez un sujet mammifère, où ladite microparticule comprend un agent chimiothérapeutique.

12. Microparticule ou composition pharmaceutique pour utilisation selon la revendication 11, où :
(i) le cancer est choisi dans le groupe constitué de : un cancer du cerveau, facultativement un glioblastome, un gliome ou un astrocytome ; un cancer des ovaires ; un cancer du foie ; et un cancer de la peau, et où ledit agent chimiothérapeutique est choisi dans le groupe constitué des : témozolomide, irinotécan, chlorotoxine, carmustine, platine(IV), platine(II), camptothécine, doxorubicine, docétaxel, maytansine, un maytansinoïde, monométhyl-auristatine E (MMAE) et un inhibiteur d'histone désacétylase (HDAC) ; ou
(ii) l'intervalle de temps entre les administrations de doses est d'au moins 1 semaine, éventuellement au moins 2, 3 ou 4 semaines, et en outre, éventuellement au moins 1, 2 ou 3 mois.

13. Article de fabrication comprenant :
une microparticule telle que définie dans l'une quelconque des revendications 1 à 8 ou une composition pharmaceutique selon la revendication 9 ;
un récipient pour loger la microparticule ou composition pharmaceutique ; et
une notice et/ou une étiquette.

14. Procédé de production d'une microparticule telle que définie dans l'une quelconque des revendications 1 à 8, le procédé comprenant :
la fourniture d'un premier liquide comprenant un soluté, un solvant et une pluralité de nanoparticules destinées à être encapsulées dans les microparticules, le soluté comprenant un polymère, la concentration de polymère dans le premier liquide étant au moins 10 % m/v, « m » étant le poids du polymère et « v » étant le volume du solvant, les nanoparticules comprenant : (i) un noyau comprenant un métal et/ou un semi-conducteur ; et (ii) une couronne comprenant une pluralité de ligands liés de façon covalente au noyau, où lesdits ligands comprennent au moins un glucide et/ou le glutathion ;
la fourniture d'un générateur de gouttelettes de liquide comprenant un composant piézoélectrique opérationnel pour générer des gouttelettes de liquide, amenant le générateur de gouttelettes de liquide à former des gouttelettes de liquide du premier liquide ;
le passage des gouttelettes de liquide à travers un gaz,
la mise en contact des gouttelettes de liquide avec un deuxième liquide de façon à amener le solvant à quitter les gouttelettes, de façon à former des microparticules solides ; la solubilité du solvant dans le deuxième liquide étant d'au moins 5 g de solvant par 100 ml de deuxième liquide, le solvant étant sensiblement miscible avec le deuxième liquide, dans lequel le deuxième liquide est fourni sous la forme d'un écoulement et le procédé comprend la mise en contact des gouttelettes de liquide avec l'écoulement de deuxième liquide.

15. Procédé selon la revendication 14, dans lequel :
(i) les nanoparticules sont fournies dans une phase mixte solvant organique:eau ; et/ou
(ii) le procédé comprend en outre la collecte des microparticules solides et la formulation ou le conditionnement des microparticules dans une composition pharmaceutique ou une forme pour administration.
